# EUROPEAN PATENT APPLICATION

(11) **EP 2 110 080 A1**
(43) Date of publication of application: **21.10.2009**
(21) Application number: 08300181.8
(22) Date of filing: 17.04.2008
(51) Int. Cl.: A61B 7/04, A61B 5/00

(54) **Electronic stethoscope**

(71) Applicant: Alcatel Lucent, 75008 Paris (FR)
(72) Inventor: Gass, Raymond, 67150, BOLSENHEIM (FR)
(74) Representative: Sciaux, Edmond

(57) **Abstract**

An electronic stethoscope comprises:
- a microphone (10),
- means (11, 13) for processing an auscultation signal supplied by the microphone (10),
- first coupling means (32) that can carry an auscultation signal from the processing means (11, 13) of the stethoscope to outer processing means (PU),
- second coupling means (33) that can carry said auscultation signal back, from the outer processing means (PU) to the processing means of the stethoscope (PU), after processing by the outer processing means,
- sound reproducing means (14), for receiving a processed auscultation sound supplied by the means for processing (11,13).

## Description

### BACKGROUND OF THE INVENTION

### Field of the invention

The invention relates to an electronic stethoscope of the type comprising a microphone, electronic sound processing means, and sound reproducing means, such as a loudspeaker or headphones. Stethoscopes are used by physicians to listen to sounds from the organism, in particular heart and lungs. An electronic stethoscope gives the possibility of active amplification and filtering to any desired degree. Furthermore there is a possibility to permit physicians to discuss the same phenomenon during simultaneous auscultation, as several headphones may well be connected to one and the same amplifier with filter.

### Description of the prior art

A known construction of an electronic stethoscope is described in U.S. Patent No. 6.134.331. It comprises: a microphone, a preamplifier, a first amplifier, a digital filtering unit, and a second amplifier coupled to an output.

The preamplifier performs impedance conversion. A pre-emphasis takes place in the first amplifier. The pre-emphasized signal is fed to the digital filtering unit. The digital filtering unit comprises an analog-to-digital converter (A/D converter), a digital filter, and a digital-to-analog converter (D/A converter). A switch selects either the filtered signal from the filtering unit, or a direct signal from the preamplifier, in order that a comparison may be made between the filtered signal and the unfiltered signal, by switching these two signals. Before such a comparison is performed, there may be loudness equalization between the two channels so that the ear will not have too great adjustment problems by the comparison. From the output amplifier, the amplified signal is brought to one or several headphones.

An electronic stethoscope can be hands-free : A wireless transmission (high frequency, low frequency, or optical) links a transducer part and a headphone part.

The output of an electronic stethoscope can be linked to a personal computer or a personal digital assistant for analyzing and displaying waveforms, for instance a phonocardiogram. Known electronic stethoscopes provide some digital audio processing, but the performances are limited by the low computing power that can be embedded in an electronic stethoscope, and by the limited possibilities of the interface for controlling the functions of the stethoscope: It can only comprise a few switches and a very small display. For instance, the US patent application n°**5.025.809** describes a digital stethoscope comprising means for detecting, digitizing, and recording a phonocardiogram. An audio processor embedded in the stethoscope locally filters the auscultation signal. Then the filtered signal is sent directly to the headset, and it may be recorded. Recorded data may be transmitted to a computer/display unit for further analysis.

Thus, there is a need to provide an electronic stethoscope that allows overcoming the performance limitations of the known electronic stethoscopes.

### SUMMARY OF THE INVENTION

The invention provides an electronic stethoscope comprising,
- a microphone,
- means for processing an auscultation signal supplied by the microphone,
- and sound reproducing means, for receiving a processed auscultation sound supplied by the means for processing;
**characterized in that** it further comprises:
- first coupling means that can carry an auscultation signal from the processing means of the stethoscope to outer processing means,
- and second coupling means that can carry said auscultation signal back, from the outer processing means to the processing means of the stethoscope, after processing by the outer processing means.

The stethoscope according to the invention benefits from an unlimited computation power for processing the auscultation sound heard by a physician, since the auscultation signal can be sent, via the first coupling means, to an auxiliary processing unit that has no limitation a priori; then it can be sent back to the stethoscope, via the second coupling means, and forwarded to the hears of the physician.

According to a preferred embodiment of the present invention,
- the first coupling means comprise a radio transmitter coupled to said microphone for sending an auscultation signal to said outer processing unit,
- and the second coupling means comprise a radio receiver coupled to said reproducing means.

According to a further aspect of the present invention, there is provided an auxiliary processing unit for boosting an electronic stethoscope,
**characterized in that** it comprises:
- first coupling means that can receive an auscultation signal from processing means of a stethoscope,
- means for processing an auscultation signal received by the first coupling means,
- and second coupling means that can send said auscultation signal back, to the processing means of said stethoscope, after processing by the means for processing.

Other features and advantages of the present invention will become more apparent from the following detailed description of embodiments of the present invention, when taken in conjunction with the accompanying drawings.

### BRIEF DESCRIPTION OF THE DRAWINGS

The invention will be described in greater detail with reference to the drawing, in which:
FIG. 1 shows a block diagram of a first embodiment of the stethoscope according to the invention.
FIG. 2 shows a more detailed block diagram of this first embodiment.
FIG. 3 shows a block diagram of a second embodiment of the stethoscope according to the invention.
If possible, like or similar reference numerals designate the same or similar components throughout the figures.

### DETAILED DESCRIPTION OF THE PREFERRED EMBODIMENTS

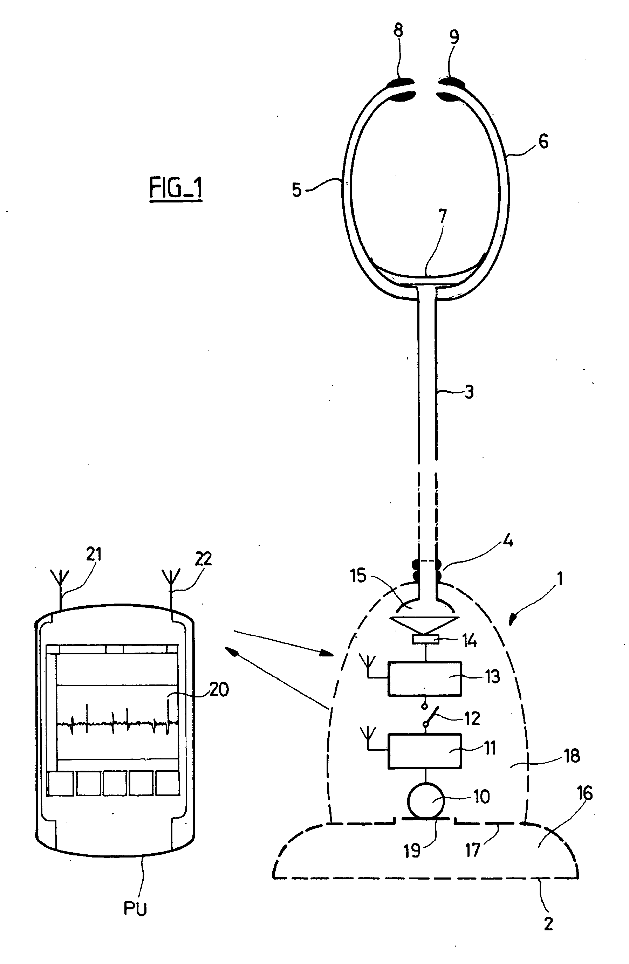

The first embodiment represented on **Fig. 1** comprises :
- A chest piece 1 that is placed on the location where the user wants to hear sound. The chest piece 1 comprises a diaphragm 2 on the bottom side.
- A conventional flexible tube 3, attached to a ribbed stem 4 of the chest piece 1.
- A conventional headset made up of two metal eartubes 5, 6, a tension spring 7, and two eartips 8, 9.
The chestpiece 1 comprises a cup shaped cavity 16 limited by the diaphragm 2 and by a metal wall 17 ; and a second cavity 18 containing an acoustical chamber 15, a loudspeaker 14, two electronic units 11-13, a switch 12, a microphone 10, and a battery not represented. The microphone 10 is placed in a hole 19 cut in the wall 17, for converting the sound transmitted by the diaphragm 2 into an electric signal.
A first electronic unit 11 amplifies the auscultation signal supplied by the microphone 10, samples it, and digitizes it. The first electronic unit 11 further comprises a Bluetooth transmitter that sends the digitized auscultation signal to an outer processing unit PU, which is a personal digital assistant for instance.
A second electronic unit 13 comprises a Bluetooth receiver that receives a digitized signal from the outer processing unit PU. This signal is the auscultation signal that has been processed by the processing unit in such a way as to provide more valuable diagnostic information. The received auscultation signal is amplified and then applied to the loudspeaker 14. The ribbed stem 4 is connected to the acoustic chamber 15 placed in front of loudspeaker 14.
This loudspeaker 14 has preferably a broad diameter (Four centimeters for instance) in order to correctly reproduce the low frequencies. The association of this broad diameter loudspeaker 14 and of the classical flexible tube 3 provides an audio transfer function that is not flat, but is similar to the transfer function of the association of the diaphragm and of the tube in a classical acoustic stethoscope. So the audio signal provided at the eartips 8, 9 sounds familiar to a physician who has been trained to auscultation with a classical stethoscope.

An auxiliary processing unit PU in real time processes the auscultation signal. A current personal digital assistant, or a personal computer, has a computation power appropriate for applying sophisticated filtering methods which need much computation power. Examples of such sophisticated filtering methods can be found in the followings documents:
- **Acoustic echo and noise control A practical approach,**
   Authors: HÄNSLER Eberhard, SCHMIDT Gerhard, WILEY Editor.
- IEEE SIGNAL PROCESSING LETTERS, VOL. 11, NO. 4, APRIL 2004,
   **A Fast Converging Algorithm for Network Echo Cancellation,**
   Mehran Nekuii, *Student Member, IEEE*, and Mojtaba Atarodi, *Member, IEEE.*
In addition, the processing unit PU processes the auscultation signal for displaying a waveform on a screen 20 of the processing unit PU. A method for such processing is described in the document US5.025.809 incorporated here by reference.
The outer processing unit PU provides a richer man-machine interface, than any electronic stethoscope. It may comprise an alphanumeric keyboard, a touch screen, a voice recognition software, etc, which are very useful means for controlling the functions of the stethoscope, and that could not be embedded in a stethoscope alone. A graphical interface displays menus in order to enable a user to switch between several transfer functions for the filtering, and several display parameters for displaying waveforms. Several sets of filter coefficients are stored in a memory of the processing unit PU.
The switch 12 enables to bypass the Bluetooth link to the outer processing unit PU. It can be used either when no processing unit PU is available, or when the physician wants to hear the auscultation signal directly, without any filtering, for comparing with the filtered signal provided by the processing unit PU.

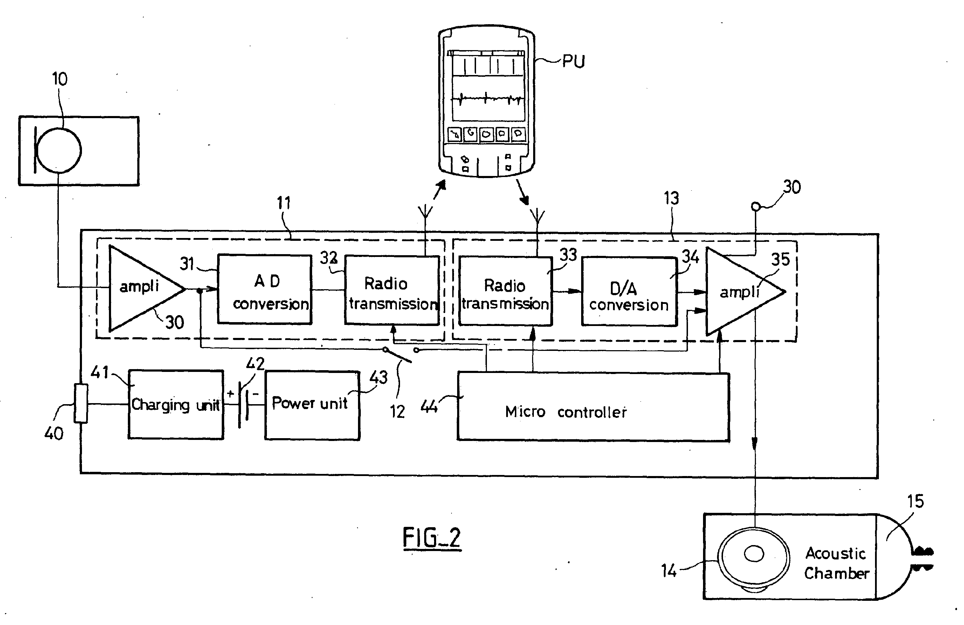

**FIG. 2** shows a more detailed block diagram of this first embodiment. The first electronic unit 11 comprises in series: an analogue preamplifier 30, an analogue-to-digital converter 31, and a Bluetooth radio transmitter 32. An input of the analogue preamplifier 30 is linked to the microphone 10. An output of the analogue preamplifier 30 is linked to an input of the Bluetooth radio transmitter 32. The output of the analogue preamplifier 30 is linked to an input of the analogue-to-digital converter 31 and to a first terminal of the switch 12. The output of the analogue-to-digital converter 31 is linked to the input of the Bluetooth radio transmitter 32.

The second electronic unit 13 comprises in series: a Bluetooth radio receiver 33, a digital-to-analogue converter 34, and an output amplifier 35. The output of Bluetooth radio receiver 33 is linked to the input of the digital-to-analogue converter 34. The output of the digital-to-analogue converter 34 is linked to a first input of the output amplifier 35. This latter has a second input linked to a second terminal of the switch 12, a first output linked to the loudspeaker 14, and a second output linked to a connector 30 where a headphone, or several headphones, can be connected.
A micro controller controls the Bluetooth radio transmitter 32, the Bluetooth radio receiver 33, and the output amplifier 35.
A rechargeable battery 42 supplies power to a power unit 43 regulating a DC voltage distributed to all the electronic components of the stethoscope. A charging unit 41 linked to a connector 40, at the surface of the housing, can charge the battery 42. When the stethoscope is not in use, an AC power adapter (not represented) is connected to the connector 40.

In a preferred embodiment, the auxiliary processing unit PU is a personal digital assistant that comprises a classical Bluetooth transmitter 21 and a classical Bluetooth receiver 22. They are associated to the classical Bluetooth transmitter 33 and the classical Bluetooth receiver 32 of the stethoscope in a classical way.
A peculiar application software is run by the personal digital assistant, which:
- enables the Bluetooth receiver 32 to receive an auscultation signal from the processing means 11, 13 of the stethoscope,
- processes the auscultation signal received by the Bluetooth receiver 32, to improve the diagnostic value of the auscultation signal, for instance by a peculiar filtering,
- and enables the Bluetooth transmitter 33 to send the auscultation signal back, to the processing means 11, 13 of the stethoscope, after processing.

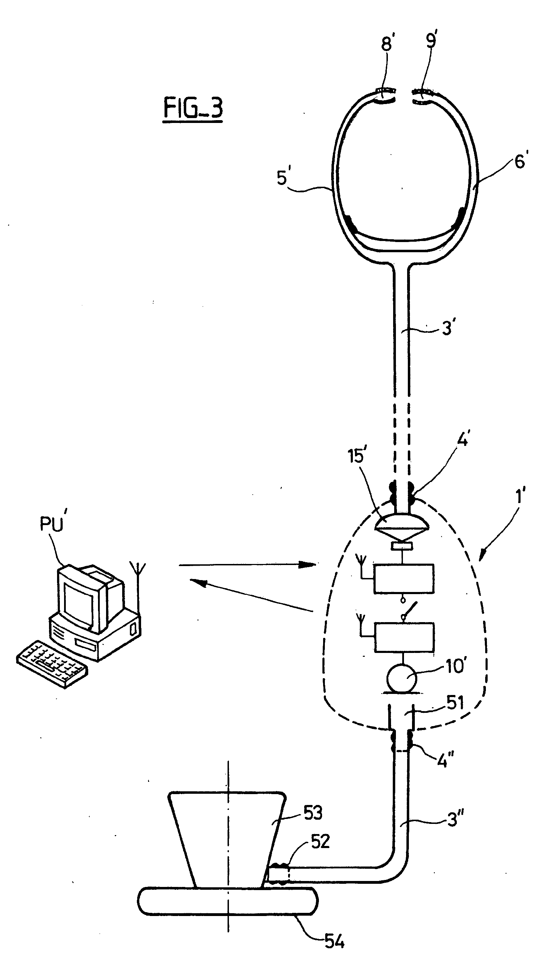

**FIG. 3** shows a block diagram of a second embodiment of the stethoscope according to the invention. This embodiment is designed to convert a classical stethoscope into an electronic one. It comprises a housing 1' with an upper ribbed stem 4' and a lower ribbed stem 4". In this example, the classical stethoscope comprised:
- A chest piece qui comprises a diaphragm 54 on one side and a bell 53 on the other side.
- A conventional flexible tube 3, attached to a ribbed stem 52 of the chest piece.
- A conventional headset made up of two metal eartubes 5', 6', a tension spring 7', and two eartips 8', 9'.
The user has converted it into an electronic stethoscope by:
- Cutting the tube 3 into two segments 3' and 3",
- Then attaching the segment 3' to the upper ribbed stem 4' of the housing 1',
- And the segment 3"to the lower ribbed stem 4" of the housing 1'.

The lower ribbed stem 4" continues into the housing 1' by a first acoustical chamber 51 containing a microphone 10'. The upper ribbed stem 4" continues into the housing 1' by a second acoustical chamber 15' containing a loudspeaker 15'.
The housing 1' also contains two electronic units 11', 13', a switch 12', and a battery not represented. These elements have respectively the same functions as the corresponding elements in the first embodiment. The auscultation signal is transmitted to an auxiliary processing unit PU' (A personal computer in this example) for filtering, and for displaying wave forms on a screen, and is transmitted back to the housing 1' for restitution by the loudspeaker 14, coupled to the tube segment 3' by the acoustical chamber 15'.

The upward and downward link with the outer processing unit preferably is wireless, however a wire could be used as well, for instance using the USB 2 technology (Universal Serial Bus version 2).

The radio transmitter 32 and the radio receiver 33 preferably use the Bluetooth technology, however they can use other wireless transmission technologies as well, for instance Zigbee or UWB.
They can use two different technologies respectively for the transmission from the stethoscope to the processing unit PU, and for the transmission from the processing unit PU to the stethoscope, these technologies being chosen in order to minimize the power consumption in the stethoscope, while providing bandwidths respectively adapted to the two signals to be transmitted.

The preferred embodiments comprise a remote loudspeaker coupled to a headset by flexible tubing, however the invention can be also applied to a stethoscope wherein the headset and tubing are replaced by headphones and a wire between the housing and the headphones.

## Claims

1. An electronic stethoscope comprising:
- a microphone (10),
- means (11, 13) for processing an auscultation signal supplied by the microphone (10),
- and sound reproducing means (14), for receiving a processed auscultation sound supplied by the means for processing (11,13); **characterized in that** it further comprises:
- first coupling means (32) that can carry an auscultation signal from the processing means (11, 13) of the stethoscope to outer processing means (PU),
- and second coupling means (33) that can carry said auscultation signal back, from the outer processing means (PU) to the processing means of the stethoscope (PU), after processing by the outer processing means.

2. An electronic stethoscope according to claim 1, **characterized in that:**
- the first coupling means (32) comprise a radio transmitter (11) coupled to said microphone (10) for sending an auscultation signal to said outer processing unit (PU) ;
- and the second coupling means (33) comprise a radio receiver (13) coupled to said reproducing means (14).

3. Auxiliary processing unit (PU) for an electronic stethoscope, **characterized in that** it comprises:
- first coupling means (32) that can receive an auscultation signal from processing means (11, 13) of a stethoscope,
- means for processing an auscultation signal received by the first coupling means,
- and second coupling means (33) that can send said auscultation signal back, to the processing means of said stethoscope (PU), after processing by the means for processing.

## Amended claims

### Amended claims in accordance with Rule 137(2) EPC.

**1.** An electronic stethoscope comprising a local unit (1-6) and an outer auxiliary processing unit (PU), said local unit (1-6) comprising:
- a microphone (10),
- first coupling means (11) that can receive an auscultation signal supplied by the microphone (10), and transmit it to the auxiliary processing unit (PU), on a first link,
- second coupling means (13) that can receive an audio signal, from the auxiliary processing unit (PU), on a second link,
- and sound reproducing means (14), for reproducing an audio signal supplied by the second coupling means (13);
said auxiliary processing unit (PU) comprising:
- receiving means (21) that can receive an auscultation signal from a local unit (1-6), on a first link,
- processing means for processing an auscultation signal received by the receiving means,
- and transmitting means (22) that can send an audio signal to a local unit (1-6), on the second link;
said auxiliary processing unit (PU) being **characterized in that** :
- the processing means are adapted for processing an auscultation signal received by the receiving means, and restituting an improved auscultation signal,
- and the transmitting means (22) are adapted to send said improved auscultation signal to a local unit (1-6), on the second link.

**2.** An electronic stethoscope according to claim 1, **characterized in that**:
- the first coupling means (11) comprise a radio transmitter (32) ;
- and the second coupling means (13) comprise a radio receiver (33).

**3.** Auxiliary processing unit (PU) for an electronic stethoscope comprising a local unit (1-6) and a remote auxiliary processing unit (PU), said auxiliary processing unit (PU) comprising:
- receiving means (21) that can receive an auscultation signal from a local unit (1-6), on a first link,
- processing means for processing an auscultation signal received by the receiving means,
- and transmitting means (22) that can send an audio signal to the local unit (1-6), on the second link;
and being **characterized in that** :
- the processing means are adapted for processing an auscultation signal received by the receiving means, and restituting an improved auscultation signal,
- and the transmitting means (22) are adapted to send said improved auscultation signal to the local unit (1-6), on the second link.

**4.** Auxiliary processing unit (PU) according to claim 3,**characterized in that**:
- the receiving means (21) comprise a radio receiver;
- and the transmitting means (22) comprise a radio transmitter.
